# EUROPÄISCHE PATENTANMELDUNG

(11) **EP 0 545 212 A1**
(43) Veröffentlichungstag der Anmeldung: **09.06.1993**
(21) Anmeldenummer: 92119937.8
(22) Anmeldetag: 24.11.1992
(51) Int. Cl.: A61K 31/57

(54) **Verwendung von Glucocorticoidmonoestern, insbesondere Dexamethason-in-Sulfobenzoat zur Herstellung von parenteral verabreichbaren Mitteln zur Behandlung von Erkrankungen, bei denen Glucocorticoide indiziert sind**

(30) Priorität: 28.11.1991 DE 4139114
(71) Anmelder: HF ARZNEIMITTELFORSCHUNG GmbH & Co. KG, D-59368 Werne (DE)
(72) Erfinder: Moormann, Jochen, Dr., W-4712 Werne (DE); Rotermund, Charlotte, Dr., W-4715 Ascheberg (DE)

(57) **Zusammenfassung**

Verwendung von Monoestern aus Glucocorticoiden einerseits und physiologisch verträglichen, mindestens eine hydrophile Gruppe tragenden organischen Säuren andererseits zur Herstellung von parenteral verabreichbaren Mitteln mit Retardwirkung, in denen sie in gelöster Form vorliegen, für mit Glucocorticoiden behandelbare Erkrankungen in der Humanmedizin, wobei die Säurekomponente als hydrophile Gruppe Hydroxyl und/oder den Sulfonsäurerest enthält.

Die Erfindung bezieht sich ferner auf ein Verfahren zur Behandlung von Erkrankungen, für die der Einsatz von Glucocorticoiden indiziert ist, bei dem man dem Patienten ein Mittel der vorgenannten Art parenteral verabreicht.

## Beschreibung

Die Erfindung bezieht sich auf die Verwendung von Glucocorticoidverbindungen zur Herstellung von Mitteln, die bei Erkrankungen, bei denen die parenterale Gabe von Glucocorticoiden indiziert ist, eingesetzt werden, sowie auf ein Verfahren zur parenteralen Behandlung von solchen Erkrankungen.

Es sind verschiedene Mittel auf der Basis von Glucocorticoiden bekannt, mit denen man z.B. Asthma bronchiale und andere Erkrankungen, wie akute entzündliche Schübe des rheumatischen Formenkreises, behandelt.

Die Einführung der Glucocorticoide in der Asthmatherapie und zur Behandlung von akuten entzündlichen Schüben des rheumatischen Formenkreises führte zu einem entscheidenden therapeutischen Fortschritt. Im akuten Asthmaanfall ist die parenterale, insbesondere intravenöse Anwendung notwendig, während die nachfolgende Behandlung mit oraler Verabreichung der Glucocorticoide und der kleinstmöglichen individuell angepaßten Dosis erfolgen sollte. Je nach der Schwere der Obstruktion tritt die volle Wirksamkeit der Glucocorticoide erst nach längerer Therapiedauer ein. Es kommt zur Erweiterung der Atemwege, zur Hemmung der Sekretion, zum Nachlassen der Hyperreagibilität, zu einem Schutz vor der Sofortreaktion nach Allergenprovokation und zur Verminderung des Anstrengungsasthmas (Schulze-Werninghaus, Debelic (Hrsg.): "Asthma - Grundlagen Diagnostik, Therapie", Springer Verlag Berlin, Heidelberg, 1988, Seite 327). Nach Rückgang der Obstruktion spricht der Patient wieder auf Beta-2-Adrenergika an, d.h. es ist eine permissive Wirkung der Glucocorticoide eingetreten.

Das geringste Risiko in der Anwendung der Glucocorticoide beim Asthma tritt bei der Behandlung mittels Inhalieren ein, jedoch ist die dadurch erzielbare Wirkung für einen akuten Asthmaanfall nicht ausreichend, ebenso wenig wie übrigens die orale.

Es kommt daher darauf an, für den akuten Asthmaanfall (Status asthmaticus) eine ausreichend schnell und ausreichend lang wirksame Darreichungsform für das Glucocorticoid zu finden. Gemäß dem jetzt bekannten Stand gilt für die parenterale (z.B. intravenöse) Behandlung des akuten Asthmaanfalls folgende Empfehlung: Gabe von 2 - 4 mg/kg Körpergewicht Prednisolon-Äquivalenten im Abstand von 4 - 6 Stunden bis zur deutlichen Verbesserung der Atemwegsfunktion (Sybrecht: "Stellenwert der Steroide beim akuten Asthmaanfall", aus Fabel (Hrsg.) "Corticosteroide bei Atemwegserkrankungen", Verlag für angewandte Wissenschaften, München, 1985, Seite 43). Die diesbezüglichen Anforderungen werden durch die bis jetzt vorhandenen Präparate nur unzureichend erreicht. Vor allem die Dauer der Wirkung nach i.v. Applikation ist oft nicht ausreichend, weshalb die i.v. Anwendung wiederholt werden muß.

Einige der Mittel auf der Basis von Glucocorticoiden zur Behandlung von Asthma bronchiale bzw. den akuten entzündlichen Schüben des rheumatischen Formenkreises oder anderen Erkrankungen, bei denen der Einsatz von Glucocorticoiden indiziert ist, sind Kombinationspräparate mit anderen Wirkstoffen; ein anderer Teil wird als Monopräparat verabreicht. So wird das Dexamethason (9-Fluor-11β,17,21-trihydroxy-16α-methyl-1,4-pregnadien-3,20-dion) als solches für die intramuskuläre Anwendung in Form eines Kombinationspräparates mit Lidocain oder für die gleiche Anwendung als Kristallsuspension in Form des Acetats, des Isonicotinats oder des Trioxaundecanoats verabreicht, und darüber hinaus in Form des m-Sulfobenzoats (d.i. 21-(3-Sulfobenzoat), Natriumsalz) als Augentropfen oder in Form des Phosphorsäureester-Natriumsalzes in injizierbarer Form als Monopräparat (vgl. Goodman and Gilman: "The Pharmacological Basis of Therapeutics", 7. Auflage, MacMillan Publishing Company, New York, 1985, S. 1476 und 1477). Aus dieser Literaturstelle ist auch bekannt, injizierbare Lösungen aus den Natriumsalzen der Bernsteinsäureester des Hydrocortisons bzw. des Prednisolons herzustellen. Diese beiden Stoffe werden in Form eines Pulvers auf den Markt gebracht, aus dem dann unmittelbar vor der Injektion die injizierbare Lösung hergestellt werden soll. Naturgemäß ist eine solche Verfahrensweise umständlich und mit Risiken behaftet, denn bei Lösungen, die in anderer Weise als intramuskulär injiziert werden, muß peinlichst darauf geachtet werden, daß keine restlichen Kristalle darin enthalten sind, auch nicht als Mikrokristalle.

Die in Deutschland geltenden gesetzlichen Bestimmungen erfordern es, daß Glucocorticoide nicht mehr in Kombinationspräparaten, sondern nur noch als Monopräparat angeboten werden dürfen, obwohl sich Kombinationspräparate bei der Behandlung des akuten Asthmaanfalls bestens bewährt hatten. Die obengenannten als Monopräparat zur Verfügung stehenden Mittel genügten jedoch aus den vorgenannten Gründen den Anforderungen der Praxis nur unvollkommen.

Von den Partialestern der Glucocorticoide ist es bekannt (siehe US-PS 3276959), daß sie relativ leicht verseifbar sind und daß wäßrige Lösungen davon zur Trübung - eine Folge von Unlöslichkeit - und Änderung des pH neigen. Daher ist es allgemein üblich, wäßrige Lösungen dieser Partialester erst kurz vor ihrer Anwendung herzustellen.

Es ist schließlich aus Untersuchungen über die intramuskuläre Verabreichung verschiedener Glucocorticoide an Pferden bekannt, daß sich diese, und zwar sowohl bei der Anwendung von Salzen als auch von Estern, wie des Acetats, des Isonicotinats, des Trioxaundecanoats, des m-Sulfobenzoats und des Phosphats (die beiden letzteren als Natriumsalze verabreicht) noch nach mehr als 4 Tagen im Urin nachweisen ließen, wobei ausdrücklich festgestellt wird, daß die Verweilzeit im Körper bei Verabreichung des Acetats, des Isonicotinats und des Trioxaundecanoats länger ist als bei Anwendung des Phosphats und des m-Sulfobenzoats (Chapman, D.I., Moss, M.S. and Whiteside, J., Vet. Record, 100: 447-450, insbesondere S. 449, linke Spalte, Abs. 2 (1977)). Hieraus war zu schließen, daß gerade die Verabreichung der Präparate in Form von Salzen gegenüber der von salzfreien Verbindungen weniger wirksam und nachteilig war.

Es wurde nun gefunden, daß sich Monoester aus Glucocorticoiden einerseits und physiologisch verträglichen organischen Säuren, die noch mindestens eine hydrophile Gruppe, und zwar Hydroxyl und/oder den Sulfonsäurerest enthalten, andererseits, durch eine bisher nicht bekannte Eigenschaft auszeichnen, nämlich über lange Zeit im menschlichen Körper zu verbleiben. Die genannten Ester sind daher hervorragend geeignet zur Herstellung von parenteral verabreichbaren Mitteln mit Retardwirkung, in denen sie in gelöster Form vorliegen, wie sie in der Humanmedizin zur Behandlung von Erkrankungen, bei denen Glucocorticoide indiziert sind, benötigt werden.

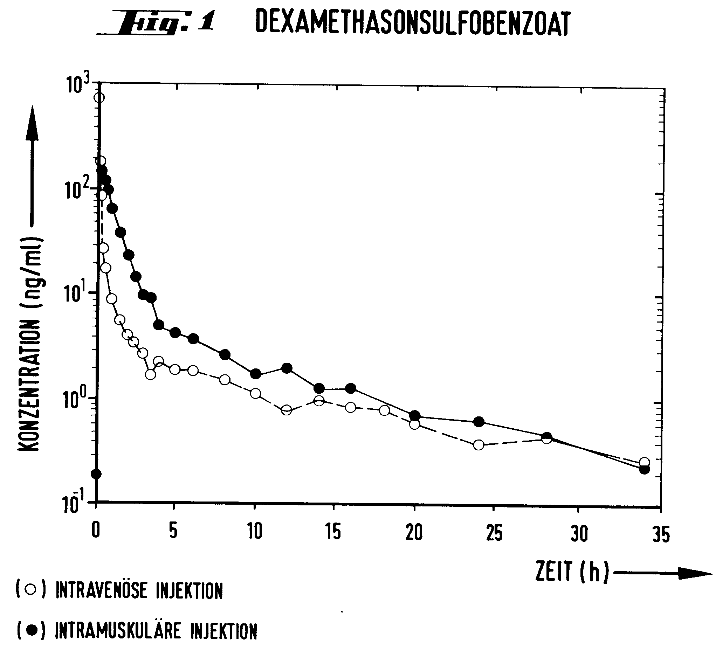

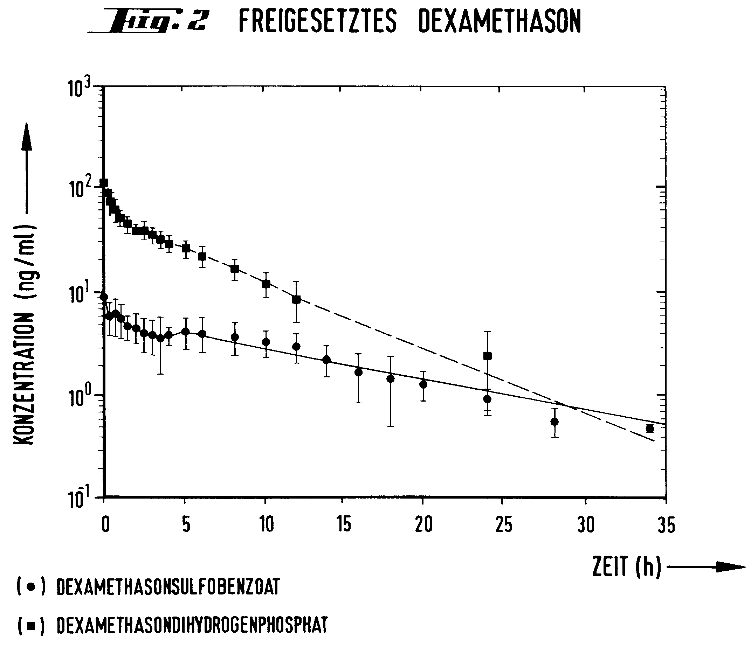

Während aus der Arbeit von Chapman et al. hervorgeht, daß sich das Dexamethasonphosphat und das Dexamethason-m-sulfobenzoat pharmakokinetisch gleichartig verhalten und dem Acetat und anderen Estern beim Pferd pharmakokinetisch unterlegen sind, wurde überraschenderweise gefunden, daß sich der Ester mit der anorganischen Phosphorsäure in der Humanmedizin gänzlich anders verhält als der Ester der organischen, wenigstens eine hydrophile Gruppe tragenden Säure. So hat sich in vergleichenden pharmakokinetischen Untersuchungen zwischen Prednisolonphosphat, Dexamethasonphosphat und Dexamethason-m-sulfobenzoat folgendes ergeben: Während das Dexamethason und das Prednisolon nach intravenöser Injektion aus den Phosphorsäureestern unmittelbar freigesetzt wird (freies Dexamethason ist unmittelbar nach Injektion in höchster Konzentration nachzuweisen und diese fällt dann exponentiell steil ab (Fig. 2)), ergibt sich für den m-Sulfobenzoesäureester ein anderes Bild. Im Plasma der Probanden ist nach intravenöser Bolusinjektion von Dexamethason-m-sulfobenzoat der Ester anfangs in den erwarteten Konzentrationen (mindestens 100 ng/ml) nachweisbar (Fig. 1), während freigesetztes Dexamethason erst mit Verzögerung und in geringeren Konzentrationen (mindestens 10 ng/ml), dafür aber über längere Zeit nachzuweisen ist (Fig. 2). Bei der intravenösen und der intramuskulären Anwendung unterscheiden sich die pharmakokinetischen Daten nicht (Fig. 1). Die Plasmakonzentration des Dexamethasons ist über mehr als 40 Stunden nahezu gleichbleibend. Die terminale Halbwertszeit (eine pharmakokinetische Kenngröße für die Bioverfügbarkeit) beträgt für das Dexamethason nach Anwendung als Sulfobenzoesäureester 10,4 Stunden, nach Gabe des Phosphats 4,6 Stunden.

Es war bisher nicht bekannt, daß sich das Dexamethason-m-sulfobenzoat und die übrigen erfindungsgemäß verwendeten Ester im menschlichen Körper derart verhalten. Überraschenderweise war diese Wirkung in den bisher üblichen Kombinationspräparaten nicht erkannt worden und auch nicht erkennbar aufgetreten. Dies dürfte darauf zurückzuführen sein, daß bei der Herstellung dieser parenteral zu verabreichenden Kombinationspräparate eine teilweise Spaltung der Ester, z. B. der Sulfobenzoate, unter den Herstellbedingungen (Sterilisationsbedingungen) und der Einwirkung der übrigen Komponenten eintritt. So wird z. B. in der US-PS 3276959 ausdrücklich darauf hingewiesen, daß die Glucocorticoidester besonders dann, wenn sie längere Zeit über 20°C gehalten werden, in Lösung nicht stabil sind. Eigene Untersuchungen haben ergeben, daß am Ende der üblichen Laufzeit solcher Kombinationsarzneimittel bereits etwa ein Drittel der Ester in Form der Einzelkomponenten vorliegt.

Die Tatsache, daß die erfindungsgemäß verwendeten Ester sich im Körper erst sehr langsam in ihre Komponenten spalten und, weil sie nicht als solche wirken, damit eine Retardwirkung ausüben, ist für die Behandlung der Erkrankungen von erheblicher therapeutischer Bedeutung. Denn dadurch wird es möglich, dem Patienten nicht mehr eine Verabreichung in bisher üblichen Zeitspannen von etwa 4 - 6 Stunden zuzumuten, sondern es kann auf eine zusätzliche Verabreichung verzichtet oder allenfalls eine einzige weitere Einzelgabe notwendig werden. Mit anderen Worten wird es mit Hilfe der Erfindung möglich, mit parenteralen Gaben gewissermaßen eine Depotform zu verabreichen. Dieses Ergebnis der längeren Wirksamkeit wird durch die gleichzeitig erhobenen Daten zur Pharmakodynamik bestätigt.

Eine Aufgabe der Erfindung besteht also demnach auch darin, Dosierungseinheiten, insbesondere in spritzfertiger Form, für die Behandlung der Erkrankungen zur Verfügung zu stellen. Derartige Dosierungseinheiten enthalten den Wirkstoff zweckmäßig in einer Menge, deren Wirkung 3 bis 6 mg Dexamethason entspricht. Für die einzelnen Glucocorticoidverbindungen ist die Menge u. a. von der sogenannten Äquivalenzdosis der Glucocorticoidkomponente abhängig. So gilt z. B. für das Dexamethason mit einer Äquivalenzdosis von 1 (relative Wirkstärke 30, bezogen auf das Hydrocortison mit 1) ein Bereich von 3-6 mg als notwendige Dosierung für den Asthmaanfall. Bei Einsatz von Methylprednisolon würden entsprechend 18-36 mg benötigt. Abhängig von der Erkrankung und abhängig von der Äquivalenzdosis der Glucocorticoidkomponente kommen andere Dosierungsbereiche in Betracht.
Die Spaltung der erfindungsgemäß verwendeten Ester verläuft im Körper über lange Zeit ziemlich gleichmäßig, ohne daß unmittelbar nach der Injektion ein Spitzenwert auftritt. Dies hat den therapeutisch sehr wichtigen Vorteil, daß das Risiko des Auftretens von Nebenwirkungen bei ausreichender Wirksamkeit deutlich vermindert wird. Derartige Nebenwirkungen bestehen bei der Kurzzeitbehandlung mittels der bisher üblichen Präparate z. B. in einer gestörten Infektabwehr, Erhöhung des Augeninnendrucks, psychischen Veränderungen sowie einer herabgesetzten Glucosetoleranz.

Bei den erfindungsgemäß erhaltenen Arzneimitteln handelt es sich um wäßrige Lösungen mit Langzeit- (Retard-) Wirkung, wobei dieser Begriff auch solche Lösungen einschließt, die noch physiologisch verträgliche Lösungsvermittler in untergeordneten Mengen, z. B. bis zu 35, vorzugsweise bis zu 30 Gewichtsprozent (bezogen auf die Lösung) enthalten, wie niedere Glykole, Glycerin und insbesondere das 1,2-Propandiol. Es kann auch zweckmäßig sein, zur Einstellung des gewünschten pH-Wertes, der natürlich im physiologisch verträglichen Bereich liegen soll, also zweckmäßig von 5 bis 8 beträgt, geeignete Mengen von neutralisierenden Substanzen, wie Natron- oder Kalilauge oder Salzsäure hinzuzugeben. Auch die Einarbeitung eines geringen Überschusses der freien Säurekomponente, z. B. Zitronen-, Wein- oder Äpfelsäure ist möglich.

Die erfindungsgemäß herzustellenden Präparate unterscheiden sich grundsätzlich von denen des Standes der Technik dadurch, daß sie retardierte, spritzfertige Lösungen darstellen. Retardierte, spritzfertige Kristallsuspensionen, z. B. Dexamethasonacetat, sind bereits bekannt. Diese haben jedoch den Nachteil, daß sie nur intramuskulär, nicht aber intravenös gespritzt (injiziert) werden können. Die erfindungsgemäß erhaltenen Mittel können aber darüber hinaus auch als Infusion, intraartikulär oder aus Depotpflastern transdermal verabreicht werden, was mit den bekannten Mitteln ebenfalls nicht möglich war. Auch eine intramuskuläre Anwendung ist natürlich möglich. Alle diese Verabreichungsformen werden im Sinne der vorliegenden Erfindung unter dem Begriff parenteral zusammengefaßt, wobei die Erfindung naturgemäß insbesondere für andere Verabreichungsformen als die intramuskuläre von Bedeutung ist, weil mit der intramuskulären Verabreichung nicht selten unerwünschte Nebenwirkungen, wie Muskelatrophien, verbunden sind.

Bei den erfindungsgemäß erhaltenen Arzneimitteln handelt es sich um Monopräparate; diese können jedoch in Einzelfällen andere Glucocorticoide wie Prednisolon enthalten, jedoch keine anderen Wirkstoffe.

Die erfindungsgemäß erhaltenen Arzneimittel sind zur Behandlung von Erkrankungen der verschiedensten Art geeignet, z. B. von Asthma bronchiale, akuten Phasen der chronischen Polyarthritis oder der Psoriasisarthritis; akutem rheumatischem Fieber; akuter Verschlechterung bei Lupus erythematodes; Arteriitis temporalis; idiopathischer thrombozytopenischer Purpura; Sarcoidose; akuter Alveolitis; Serumkrankheit; Transfusionszwischenfällen; Zöliakie (glutenbedingte Enteropathie); schwerer Überempfindlichkeit bei Insektenstich; anaphylaktischem Schock; angioneurotischer Ödeme (z. B. Quincke-Ödem); Cerebral-Ödemen, die mit Neoplasmen assoziiert sind; Pseudo-Krupp (besonders im Kindesalter); akuten schweren Dermatosen; akuten Komplikationen bei Colitis ulcerosa (sog. toxischem Colon). Ein besonders wichtiges Anwendungsgebiet besteht in der Behandlung von akuten Schüben des rheumatischen Formenkreises.

Als Glucocorticoidkomponente der Ester kommen übliche Stoffe (Steroidalkohole) in Frage, wie das körpereigene Cortisol und Derivate davon wie Corticosteron, 11-Dehydrocorticosteron, 17-Hydrocorticosteron, Cortison, Fluorhydrocortison, 1-Dehydrocortison, Prednisolon, Testosteron, fluorierte Methylprednisolone wie das Dexamethason, Betamethason oder das Flumethason. Als Säurekomponente kommen Reste von physiologisch verträglichen organischen Säuren in Betracht, die außer der esterartig gebundenen Säuregruppe noch zusätzlich eine und zweckmäßig höchstens zwei hydrophile Gruppen enthalten können, wie SO₃H-, COOH- oder OH-Gruppen. Geeignete Reste sind z. B. der Sulfobenzoat-, insbesondere der m-Sulfobenzoatrest, ferner der Monoweinsäure-, Monoäpfelsäure- und Monozitronensäurerest. Der Sulfobenzoatrest ist vorteilhaft unsubstituiert, kann aber auch substituiert sein.

Dann enthält er zweckmäßig am Benzolrest nicht mehr als zwei weitere Substituenten, und zwar einen Alkylrest mit 1 bis 2 C-Atomen oder einen weiteren Hydroxyl- oder Sulfonsäurerest oder eine Kombination davon. Alle diese Verbindungen werden bevorzugt in Form ihrer wasserlöslichen physiologisch verträglichen Salze, insbesondere eines Alkali- und hiervon des Natriumsalzes verabreicht.

Die pharmakokinetischen Eigenschaften der erfindungsgemäßen Arzneimittel sind aus den beiden nachstehenden Graphiken (Fig. 1 und Fig. 2) ersichtlich. Die Werte sind in der graphischen Darstellung in halblogarithmischem Maßstab dargestellt, woraus der Fachmann die Effekte deutlich erkennen kann. Figur 1 zeigt die Plasmakonzentration (Mittelwert von 12 Probanden) für Dexamethasonsulfobenzoat nach intravenöser (o) bzw. intramuskulärer (●) Injektion von 9,1 mg Dexamethasonsulfobenzoat (entspricht 6 mg Dexamethason); Figur 2 zeigt die Plasmakonzentration (Mittelwert von 12 Probanden plus/minus Standardabweichung) von freigesetztem Dexamethason nach intravenöser Injektion von 9,1 mg Dexamethasonsulfobenzoat (●) (entspricht 6 mg Dexamethason) bzw. 6,6 mg Dexamethasondihydrogenphosphat (■) (entspricht 6 mg Dexamethason).

### Beispiel

Es wird eine Ampulle hergestellt, die 5 ml einer wäßrigen Injektionslösung enthält. Darin sind 9,15 mg des Natriumsalzes des Dexamethason-21-(3-sulfobenzoats) enthalten, welche Menge 6 mg Dexamethason entspricht. Zur Verbesserung der Löslichkeit des Wirkstoffs enthält die Lösung 30 % (m/v) 1,2-Propandiol.

Bei der Anwendung wird die Dosierung individuell dem Krankheitsgeschehen angepaßt; in der Regel werden bei akuten und bedrohlichen Situationen, z. B. schweren Asthmaanfällen und ähnlichen Zuständen bei chronischer Bronchitis anfangs 1-2 Ampullen langsam intravenös verabreicht. Diese Dosierung reicht in der Regel aus, so daß eine weitere Behandlung danach nicht mehr erforderlich ist.

## Patentansprüche

1. Verwendung von Monoestern aus Glucocorticoiden einerseits und physiologisch verträglichen, mindestens eine hydrophile Gruppe tragenden organischen Säuren andererseits zur Herstellung von parenteral verabreichbaren Mitteln mit Retardwirkung, in denen sie in gelöster Form vorliegen, für mit Glucocorticoiden behandelbaren Erkrankungen in der Humanmedizin, wobei die Säurekomponente als hydrophile Gruppe Hydroxyl und/oder den Sulfonsäurerest enthält.

2. Ausführungsform nach Anspruch 1, dadurch gekennzeichnet, daß das Mittel zur Behandlung von Asthma bronchiale bestimmt ist.

3. Ausführungsform nach Anspruch 1, dadurch gekennzeichnet, daß das Mittel zur Behandlung von akuten Schüben des rheumatischen Formenkreises bestimmt ist.

4. Ausführungsform nach einem oder mehreren der Ansprüche 1 - 3, dadurch gekennzeichnet, daß die physiologisch verträgliche Säurekomponente noch zusätzlich eine und zweckmäßig höchstens zwei hydrophile Gruppen enthält und vorzugsweise der Sulfobenzoatrest ist, der am Benzolrest noch höchstens zwei weitere Substituenten, und zwar einen Alkylrest mit bis zu zwei C-Atomen oder einen weiteren Hydroxyl- oder Sulfonsäurerest oder eine Kombination davon tragen kann, oder ein einbasisches Salz der Wein-, Äpfel- oder einfach veresterten Zitronensäure ist.

5. Ausführungsform nach einem oder mehreren der Ansprüche 1 - 4, dadurch gekennzeichnet, daß der Monoester in Form eines physiologisch verträglichen Salzes, vorzugsweise eines Alkalisalzes und insbesondere des Natriumsalzes vorliegt, wobei vorzugsweise die Corticoidkomponente das Dexamethason und der Wirkstoff vorzugsweise ein Alkalisalz des Dexamethason-m-sulfobenzoats und insbesondere das Natriumsalz davon ist.

6. Ausführungsform nach einem oder mehreren der Ansprüche 1 - 5, dadurch gekennzeichnet, daß das Mittel in Form einer reinwäßrigen Lösung oder einer wäßrigen Lösung, die noch physiologisch verträgliche Lösungsvermittler in untergeordneten Mengen enthält, vorliegt, wobei der Lösungsvermittler vorteilhaft in einer Menge von bis zu 35, insbesondere von bis zu 30 Gewichtsprozent, bezogen auf die Lösung, enthalten ist und das Mittel vorzugsweise einen pH-Wert zwischen 5 und 8 hat.

7. Ausführungsform nach einem oder mehreren der Ansprüche 1 - 6, dadurch gekennzeichnet, daß die Monoester zur Herstellung von Mitteln verwendet werden, die zu einer anderen parenteralen Verabreichung als der intramuskulären bestimmt sind.

8. Ausführungsform nach einem oder mehreren der Ansprüche 1 - 7, dadurch gekennzeichnet, daß das Mittel in spritzfertiger Form vorliegt.

9. Ausführungsform nach einem oder mehreren der Ansprüche 1 - 8, dadurch gekennzeichnet, daß das Mittel in Dosierungseinheiten vorliegt, von denen jede den Ester in einer Menge enthält, deren Wirkung einem Gehalt von 3 bis 6 mg Dexamethason entspricht.

10. Verfahren zur Behandlung von Erkrankungen, für die der Einsatz von Glucocorticoiden indiziert ist, dadurch gekennzeichnet, daß man dem Patienten ein Mittel gemäß einem oder mehreren der Ansprüche 1 - 9 parenteral verabreicht.
